# EUROPEAN PATENT SPECIFICATION

(11) **EP 4 192 350 B1**
(45) Date of publication and mention of the grant of the patent: **16.09.2026**
(21) Application number: 21853704.1
(22) Date of filing: 09.08.2021
(51) Int. Cl.: A61B 5/1455, A61B 5/00, G01N 33/48, G01N 33/53

(54) **WEARABLE SPECTROMETER FOR BIOMOLECULE INTERROGATION IN BIOLOGICAL TISSUE, AND METHOD**
TRAGBARES SPEKTROMETER ZUR BIOMOLEKÜLABFRAGE IN BIOLOGISCHEM GEWEBE, UND VERFAHREN
SPECTROMÈTRE PORTATIF POUR L'INTERROGATION DE BIOMOLÉCULES DANS UN TISSU BIOLOGIQUE, ET PROCEDÉ

(30) Priority: 07.08.2020 US 202063062478 P
(43) Date of publication of application: 14.06.2023
(73) Proprietor: Molecular Photonix LLC, Lutz, FL 33549 (US)
(72) Inventor: CUCINELLI, Nicholas, S., Ann Arbor, MI 48105 (US); ORAIQAT, Ibrahim, Ann Arbor, MI 48108 (US); CLARKE, Roy, Ann Arbor, MI 48103 (US); KONICZEK, Martin, Ann Arbor, MI 48104 (US)
(74) Representative: Lewis Silkin LLP
(86) International application number: PCT/US2021/045166
(87) International publication number: WO 2022/032218

(56) References cited:
- WO-A1-2017/123926
- WO-A1-2018/208775
- AU-A1- 2014 200 664
- CN-A- 108 937 955
- GB-A- 2 489 717
- US-A1- 2007 004 975
- US-A1- 2015 025 340
- US-A1- 2016 238 440
- US-A1- 2016 327 476
- US-A1- 2016 356 720
- US-A1- 2017 014 056
- US-A1- 2020 124 535
- WU GANG ET AL: "Miniaturized Computational Spectrometer", IEEE NANOTECHNOLOGY MAGAZINE, IEEE, USA, vol. 17, no. 6, 1 December 2023 (2023-12-01), pages 36 - 42, XP011953663, ISSN: 1932-4510, [retrieved on 20231018], DOI: 10.1109/MNANO.2023.3316870
- SAHA SREENIL ET AL: "Wearable SiPM-Based NIRS Interface Integrated With Pulsed Laser Source", IEEE TRANSACTIONS ON BIOMEDICAL CIRCUITS AND SYSTEMS, IEEE, US, vol. 13, no. 6, 1 December 2019 (2019-12-01), pages 1313 - 1323, XP011763655, ISSN: 1932-4545, [retrieved on 20200101], DOI: 10.1109/TBCAS.2019.2951539

## Description

### CROSS REFERENCE TO RELATED APPLICATIONS

This application claims priority to Provisional Patent Application US 63/062,478 filed on August 7, 2020.

### BACKGROUND OF THE INVENTION

Field of the Invention. The invention relates generally to a wearable miniaturized spectrometer apparatus suitable for non-invasive interrogation of subcutaneous molecules in biological tissue.

Description of Related Art. Diabetes mellitus is a chronic, incurable metabolic disease in which the body fails to produce sufficient insulin and therefore cannot control the concentration of glucose in the blood. Currently there are over a 100 million diabetics worldwide and the World Health Organization expects this will exceed 400 million during the next decade. Diabetes is diagnosed and managed by measurement of blood sugar levels. Diabetics need to closely monitor and control their glucose levels and measure them several times a day. The current approach, in place for decades, uses small blood volumes, typically extracted from the finger, to measure glucose. Repeated drawing of blood through these finger sticks is painful, presents the risk of infection, and is a major cause of treatment noncompliance. Alternative sensor devices that would allow clinicians and patients to measure glucose levels precisely, without the discomfort of extracting blood samples, are not yet widely implemented owing to the complexity and expense of the instrumentation required. An international panel of experts recently concluded that a real time continuous glucose monitor (CGM) would be a major step forward for diabetes management if the technical challenges can be overcome.

Effective treatment of diabetes requires frequent (and ideally continuous) monitoring of blood glucose levels to enable the patient to maintain basic health and avoid potentially life-threatening events such as hypoglycemia, stroke, or heart attack. For decades, the state-of-the-art in glucose monitoring has been a handheld electrochemical device that uses a physical blood sample obtained by an inconvenient and painful finger prick. More recently, so-called minimally invasive technologies have been introduced which use remote transmitters and subcutaneous probes/patches (microdermal needles, wires inserted beneath the skin, or other implantable probes) to collect real time blood glucose data and send it to a smart phone or other receiver. These approaches are limited by the quality of the probes/sensors, signal interference from interstitial fluid and tissue structures, and the resultant need for sophisticated signal analysis to indirectly approximate actual blood glucose levels. The high cost of disposable probes is a further challenge. The next and final step is a truly noninvasive blood glucose meter which increases patient comfort and compliance, delivers accurate, real time, actionable data, and can be readily integrated into the latest wearable smartwatch platforms.

Many university and industry teams are now pursuing the goal of a noninvasive glucose meter and have published studies involving lasers, fiber optics, millimeter wave, ultrasound, tabletop confocal Raman spectrometers, and even biofunctional nanoparticles injected into the patient. These studies have validated that transcutaneous spectroscopic approaches can be as effective as a finger prick test, but they are fundamentally challenged by the need for bulky and expensive spectrometers and other equipment to detect a limited signal in a complex and dynamic environment, and not readily suited to low-cost, noninvasive, wearable applications.

Patient dissatisfaction with the functional lifetime of the disposable sensors (typically 5-14 days), sensor penetration of the skin, discomfort related to patch/transmitter placement and protrusion from the body, and the long-term costs of monitoring is widespread. Such dissatisfaction is particularly intense for type 2 patients who do not see the cost/benefit value of continuous monitoring.

More importantly, current solutions still require frequent calibration checks; the approaches may be minimally invasive in the sense that they minimally penetrate the body during their measurement process, but because they still require frequent direct measurement of blood glucose levels for calibration and safety from the perspective of the end customer/patient, they are most definitely not. At this time, a 100% noninvasive and pain-free technology to monitor blood glucose in the wearable format of the real time CGM does not exist. Furthermore, current technologies tend to be indirect measurement approaches that imply direct measurement but actually employ artificial intelligence/AI to typically yield lagging, quantitatively suspect data.

There have been many ingenious attempts to develop a truly noninvasive CGM since the first CGM was introduced by MiniMed/Medtronic in 1999, but to date there is no such device on the market or even close to commercialization. As a result of this shortcoming in commercially available options, diabetic patients must manage their glucose levels using subcutaneous implanted probes which need to be replaced every few days or weeks, or in the case of one device (only approved in the EU), every few months. Even if minimally invasive, in most cases the current CGM devices still require frequent calibrations by painful finger sticks on a daily basis. Thus far, no truly noninvasive CGM has received FDA approval, primarily because of poor sensitivity, especially at the lower end of the diabetic spectrum (< 70 mg/dL), and also lack of specificity. The latter issue is particularly limiting since many of the methods put forward for noninvasive probes do not directly measure a specific correlation with the concentration of glucose in the blood or interstitial fluid. In fact, almost every physiological parameter of the body shows some correlation with oral glucose intake, and hence prior attempts at noninvasive probing of blood glucose (e.g. IR transmission/ absorption, acoustic coupling, tissue optical reflectivity, microwave response, etc.) are typically too confounded by multiple physiological tissue responses to be reliable quantitative measures of actual blood glucose levels. Thus, it is critical to find a way to couple the sensor probe directly to the glucose molecule itself. Unfortunately, there are very few noninvasive probe approaches that could do this directly, and even fewer which can be sufficiently miniaturized in a wearable device.

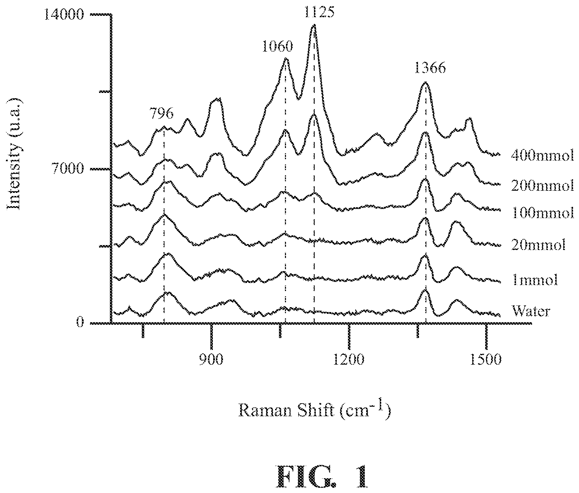

Raman spectroscopy is an optical technique that is able to directly sense the unique vibrational fingerprint frequencies of the glucose molecule. In theory, Raman spectroscopy, offering millimolar sensitivity, is able to provide accurate measurements over the 4-10 mmol/L range of physiological concentration of glucose. See for example Figure 1, which is reprinted from Jingwei Shao, et al., In Vivo Blood Glucose Quantification Using Raman Spectroscopy, PLoS ONE 7, e48127 (October 2012). DOI:10.1371/journal.pone.0048127 SourcePubMed. However, there are several challenges to the practical application of Raman spectroscopy to the field of CGM. One is the low cross-section (~1 in 10⁷) of Raman scattering. Another is the presence of non-glucose related scattering resulting from the complex chemical nature of the blood-tissue matrix in which the Raman signal is being generated. Finally, in terms of commercialization of a practical/wearable CGM device, the bulky volume and geometry of conventional Raman spectroscopy instrumentation does not lend itself to miniaturization.

US20200107756A1 describes a silicon photomultiplier (SiPM) array-based multispectral optical probe for image-guided radiotherapy used for low light detection of Cerenkov Emission (CE) in connection with tumor detection and therapy. However, there is no known system integration of this technology to accomplish a wearable, real-time, continuous diagnostic spectrometer apparatus. GB 2 489 717 discloses a wearable Raman spectrograph.

There is therefore a need in the art for a non-invasive, wearable, real-time, continuous diagnostic spectrometer apparatus and method useful to diagnose certain conditions of interest present in subcutaneous biological tissue. There exists a need for devices and methods to painlessly interrogate subcutaneous molecules that are capable of producing highly accurate and reliable information on a continuous basis. Raman spectroscopy is promising, however there is no known method to implement Raman spectroscopy in a miniature, wearable format.

### BRIEF SUMMARY OF THE INVENTION

A first aspect of this invention is defined by claim 1. A non-invasive, wearable, diagnostic spectrometer apparatus includes a housing. The housing has a sidewall surrounding an interior cavity. A cover is disposed over the sidewall and the interior cavity. A base is disposed under the sidewall at least partially enclosing the interior cavity. The base has a port therein configured to enable the transit of optical photons therethrough. Lashing extends from the housing and is configured to immobilize the housing against the skin of a user at a region of interest. A light source is configured to emit photons of light through the port that will probe physiological biomarkers in molecules of interest in subcutaneous tissue and return photons of Rayleigh scattered light commingled with Raman scattered light through the port and into the internal cavity. An array of photodetectors are disposed in the housing. Each photodetector comprises a discrete channel configured to detect photons of Raman scattered light entering the cavity through the port. At least one optical filter is associated with each photodetector. The at least one optical filter is operatively disposed between the associated the photodetector and the port. Each optical filter limits the transit of light reaching the associated the photodetector to a specific wavelength and eliminates Rayleigh scattered light. A data acquisition electronics module is operatively associated with the array of photodetectors. The data acquisition electronics module is configured to count single photons of Raman scattered light reaching each photodetector over a predetermined sampling time.

The invention also contemplates a method for non-invasively diagnosing a condition of subcutaneous biological tissue using Raman spectroscopy. The method comprises a series of steps, which include stationing a plurality of photodetectors in an internal cavity. The interior cavity is immobilized directly against the skin of a user. Light is emitted from a light source in the interior cavity through a port and directly onto the skin of the user. The light is used to interrogate at least one subcutaneous molecule below the skin of the user. The interrogating step produces optical photons of Rayleigh scattered light commingled with Raman scattered light that re-enter the internal cavity through the port. The plurality of photodetectors are shielded inside the internal cavity from the light emitted by the light source but not from the Raman scattered light re-entering the internal cavity through the port. Rayleigh scattered light is eliminated from the photons re-entering the internal cavity through the port, such as by means of a narrow-band optical filter. And the Raman scattered light reaching each photodetector is limited to a specific wavelength associated with a Raman-active spectral line. And finally, single photons received in each photodetector are counted over a predetermined sampling time to measure the integrated intensity of the selected Raman active line.

In both apparatus and method forms, this invention enables a truly noninvasive, painless, wearable, real time Raman spectroscopy diagnostic tool to drastically improve the lives of users and enhance health outcomes. The multi-spectral probe architecture is readily adapted to a variety of conditions, such as reading blood sugar and other heath parameters of interest in a wearable form factor. The invention utilizes the faint Raman-scattered light transmitted through tissue (following interrogation by a self-contained light source) to enable spectral measurements of relevant molecular biomarkers. Inelastically scattered light characteristic of Raman spectroscopy is weak, leaving only a handful of measurable photons carrying information of interest, and only a subset of these photons within specific frequencies are useful for molecular measurements. Employing Raman spectroscopy, the present invention is useful to target the tiny fraction of scattered photons that carry the fingerprint and concentration of the molecules of interest. By eliminating the need for a physically large and expensive spectrometer, the invention enables a compact, wearable, and low-cost, low-power device and method that directly and noninvasively measures molecular biomarkers of interest.

### BRIEF DESCRIPTION OF THE SEVERAL VIEWS OF THE DRAWINGS

These and other features and advantages of the present invention will become more readily appreciated when considered in connection with the following detailed description and appended drawings, wherein:
Figure 1 is a graph, reprinted from Jingwei Shao, et al., *In Vivo Blood Glucose Quantification Using Raman Spectroscopy,* depicting unique vibrational fingerprint frequencies of the glucose molecule;
Figure 2A is an example of a wearable device according to this invention in the wristband form factor;
Figure 2B is an example of a wearable device according to this invention in the fingertip clamp form factor;
Figure 2C is an example of a wearable device according to this invention in the adhesive patch form factor;
Figure 2D is an example of a wearable device according to this invention in the earlobe clip form factor;
Figure 3 is a simplified fragmentary side view of the wearable device according to this invention in the wristband form factor similar to that shown in Figure 2A and configured for wired and wireless communication with remote computing and/or remedial devices;
Figure 4 is a bottom view of the wearable device taken generally along lines 4-4 in Figure 3;
Figure 5 is a cross-sectional view of the wearable device taken generally along lines 5-5 in Figure 4;
Figure 6 is a view as in Figure 5 showing the wearable device secured in an operative position against skin of a user at a region of interest, with a molecule of interest being located in subcutaneous tissue; and
Figure 7 is a view as in Figure 6 showing photons of light emitted through the port and interrogating the molecule of interest, with photons of Rayleigh scattered light commingled with Raman scattered light returning into the internal cavity through the port.

### DETAILED DESCRIPTION OF THE INVENTION

The invention describes a non-invasive, wearable, real-time, continuous diagnostic spectrometer apparatus and method of use by which a condition of interest present in subcutaneous biological tissue can be diagnosed and monitored. The invention interrogates subcutaneous molecules at a location of interest using a painless technique capable of producing highly accurate and reliable information on a continual basis. Many deep-tissue measurement applications of the present invention are contemplated, including but not limited to: continuous glucose monitoring (CGM), toxicology screening, cancer cell detection, tumor pH, oxygenation, radiation dosage during cancer radiotherapy, proteins (troponin) associated with myocardial infarction risks, and the like. In other words, the wearable device of this invention is broadly understood as a spectroscopic sensor platform for a wide range of biomolecular interrogations. The wearable device can be used to probe physiological biomarkers in molecules of interest wherever they may reside -- in the blood, skin, tissue lipid membrane layers, etc.

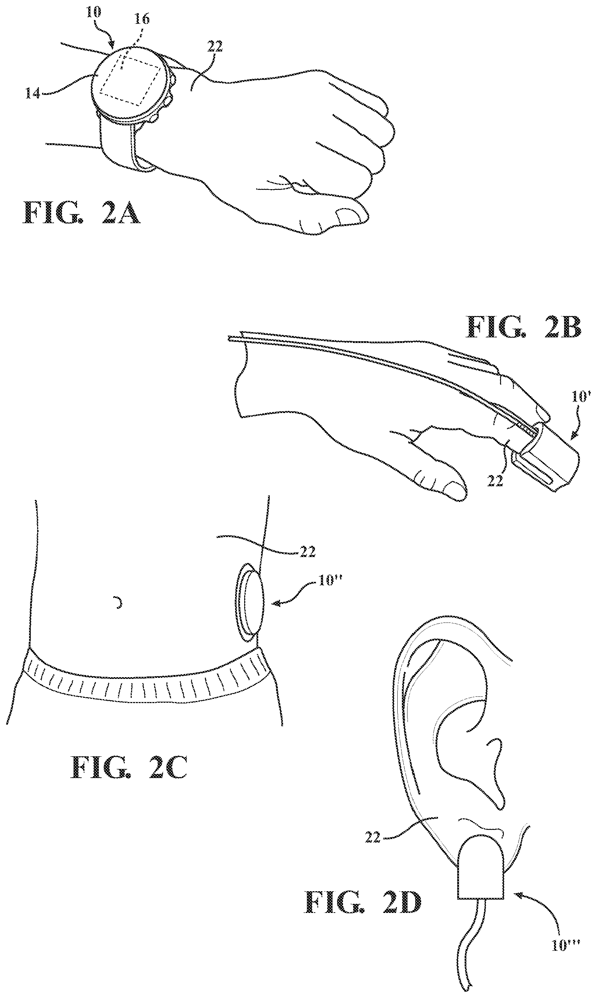

As a wearable device, the invention can manifest in different forms. Some examples are depicted in Figures 2A-D. In Figure 2A, the wearable device 10 is shown in the form of an electronic bracelet similar in appearance to a smart watch. In Figure 2B, the wearable device, generally indicated at 10', is shown in the form of a fingertip sensor. In Figure 2C, the wearable device 10" is depicted as a dermal patch. And in Figure 2D, the wearable device 10‴ is in the form of an ear lobe clip. Those of skill in the art will appreciate other forms of wearable medical sensors that can be modified or adapted for use in connection with the present invention, including configurations worn on or around the head, neck, shoulders, arms, hands, chest, waist, legs, ankles, feet, and so forth.

For convenience, the wearable device 10 will be described in an exemplary wrist-worn form like that of Figure 2A. However, it must be understood that the invention is not limited to wrist-worn forms. It is contemplated that any of the forms mentioned, as well as those not mentioned but otherwise readily appreciated by those of skill in the art, could be suitable alternative forms within which to implement the invention.

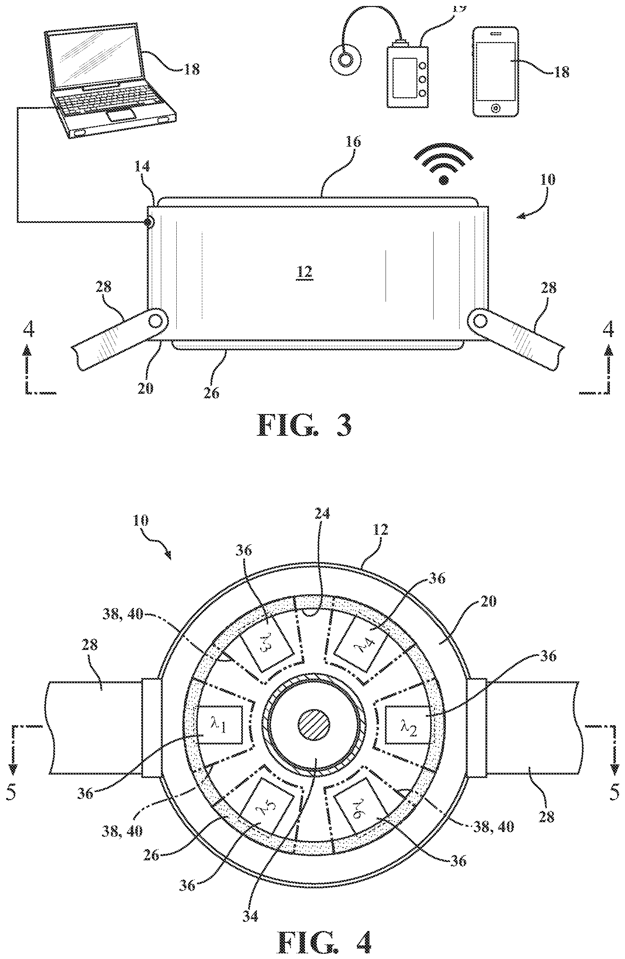

Referring to the example of Figures 3-5, the wearable device 10 includes a housing (or multiple housings) containing sensitive electronic equipment and optical hardware. The housing has a sidewall 12 surrounding an interior cavity. In the illustrated examples, the sidewall 12 is generally circular or cylindrical. However, in other contemplated embodiments the sidewall 12 can be another geometric shape or asymmetric. A cover 14 is disposed over the sidewall 12 and over the interior cavity. The cover 14 is typically the uppermost or outermost visible feature of the wearable device 10. In some embodiments, a display screen or graphic user interface (GUI) 16 may be fitted to the exterior side of the cover 14. For example, a simple display screen 16 might display information in the form of text, images, video, graphs, and the like. Configured as a GUI 16, inputs could also be received by the user to change settings or attributes of the wearable device, send and receive data, and the like. Although not shown, a speaker may be included with the wearable device 10 to provide audible messages, or tones/beeps that will communicate relevant information or alerts to the user. Likewise, additional user interface elements, such as buttons, LEDs, dials, and/or touch-sensitive elements could be placed on the housing or some other feature of the wearable device 10.

As shown schematically in Figure 3, the wearable device 10 may be configured to communicate with remote computing devices 18 via wired and/or wireless connections. It is contemplated that the wearable device 10 may be equipped with suitable data transmitting/receiving capabilities that will enable connection to the internet, World Wide Web, or other desired network. Naturally, precautions will be implemented to assure secure transmission of data to and from the wearable device 10 so that only the user and other authorized individuals may access the wearable device 10 remotely. The ability to communicate with remote computing devices 18 provides the user, the user's caregivers and other authorized individuals the ability to monitor diagnostic information generated by, and manage operation of, the wearable device 10. Furthermore, notifications can be sent to and from the user and/or the user's caregiver, and/or authorized healthcare professionals via a remote computing device 18. Such notifications can be derived from measurements taken by the wearable device 10 and/or alarms triggered when predetermined conditions are met. Moreover, data can be sent to and received from the controller 19 of a remote remedial device, such as a pump for insulin or other medication substances, a defibrillator (wearable or implanted), a brain stimulator, or the like.

A base 20 is disposed under the sidewall 12 and at least partially closing the interior cavity. In many cases, including the one depicted in Figures 3-7, the base 20 is intended to press directly against the skin 22 of a user, directly over a location of interest where diagnostic interrogation of subcutaneous biological tissue resides. In the case of a wrist-worn device 10 like that shown in Figures 3-7, the subcutaneous biological tissue to be interrogated must reside in the immediate vicinity of the wrist. Furthermore, the base 20 can be contoured for comfort according to the intended application. In the example of 2B, the base (not easily seen) would be shaped to cradle the human finger.

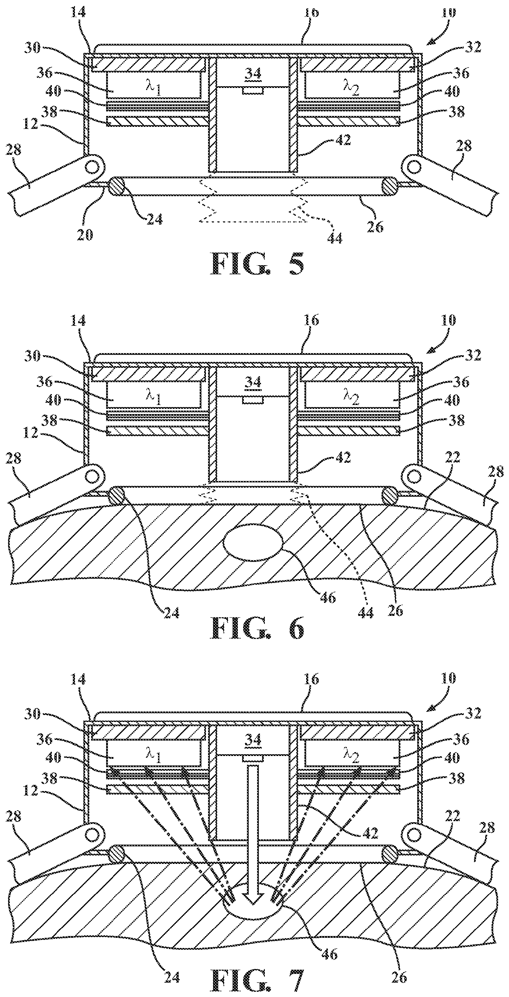

The base 20 is shown in Figures 4 and 5 having a port 24 formed therein. The port 24 is configured to enable the transit of optical photons. That is to say, optical photons are transmissible through the port 24, as suggested in Figure 7. Such transmissibility of optical photons can take different forms. In some contemplated examples, the port 24 is a membrane or pane-like element through which desired optical photons may pass freely, perhaps filtering unwanted light (e.g., Rayleigh scattered light) or other electromagnetic signals. In the illustrated examples, however, the port 24 comprises an uncovered aperture thus enabling direct movement of optical photons between the interior cavity and the underlying skin 22 of the user.

The port 24 may be any suitable shape. In the embodiment shown in Figure 4, the port 24 is generally circular, however other shapes are certainly possible. The port 24 may also be composed of a plurality of discrete smaller ports rather than one large opening. The accompanying illustrations show an elastomeric gasket 26 surrounding the port 24. The primary function of the gasket 26 could be to perfect a light-blocking seal between the base 20 and the user's skin 22 directly under the internal cavity, or simply to improve comfort. Alternatively, the base 20 could be made flexible so as to self-conform against the contours of the user's skin 22, and thus achieve the desired light-blocking and/or comfort objectives.

Some form of lashing 28 is configured to immobilize the housing against the skin 22 of a user over a region of interest. Of course, the lashing 28 can take many different forms to suit the desired function and/or style. In the example of Figures 2A and 3-7, lashing 28 comprises a wrist band of the types used to secure wristwatches and bracelets. The example of Figure 2C shows the lashing in the form of an adhesive. In Figures 2B and 2C, the lashing comprises a spring clamp.

Indeed, many options are available for the lashing based largely on the intended application. One need only look to present accommodations used for wearables on or around the head, neck, shoulders, arms, hands, chest, waist, legs, ankles and feet to gain inspiration for a lashing configuration suitable for use with the wearable device 10. Note also that the lashing 28 could also contain additional elements or sensors for user interface, communication, power, measurement, light source, and so forth.

Components contained within the internal cavity of the housing will be described presently in reference primarily to Figures 4 and 5. At some suitable location in or on the housing, in or on the lashing 28, or otherwise operatively associated with the wearable device 10 will be a power source 30. The power source 30 is shown disposed inside the interior cavity, however any location that is operatively associated with the wearable device 10 may suffice. The power source 30 may comprise any suitable electrical energy storage and delivery devices, including but not limited to batteries, beta-voltaic power sources, supercapacitors, fuel cells, wireless power, solar cells, energy harvesters and the like. The power source 30 is operatively connected to a circuit board 32, or otherwise integrated into an operating system with which the electronics of the wearable device 10 are powered and controlled.

A light source 34 is configured to emit light through the port 24. The light source 34 is disposed in the interior cavity. The light source 34 useful for this invention must be capable of producing optical photons of relevant character when interacted with subcutaneous biological tissue. That is to say, the light source 34 can activate multiple responses from the tissue when probed, including but not limited to Raman scattering, infra-red emission, fluorescence and phosphorescence. A light source suitable for use in connection with the wearable device will produce light in the spectral band of about 200 nm - 1500 nm. The light source 34 is configured to produce monochromatic light. In another unclaimed embodiment, the light source 34 is configured to produce broadband light. Thus, light sources 34 for the wearable device 10 are light emitting diodes. Other types of suitable light sources 34 may also be possible but are not within the scope of this invention. The light source 34 comprises monochromatic LEDs that will excite normal modes of vibration in molecules of interest, with specific frequency in the near UV-VIS- IR spectral band (200nm - 1500nm) chosen to be resonant or non-resonant with selected excitation modes of interest.

In some applications, it may be desirable to configure the light source 34 as capable of being modulated, with a duty cycle over which optical photons can be counted. The optical photons can be counted over the duty cycle when the light source is turned on. Or alternatively the optical photons can be counted over the duty cycle when the light source is turned off (allowing for a background measurement). The measurement signal is determined as the difference between the optical photons counted when the light source is on and when the light source is off. Notably, the light source 34 of this invention can be distinguished from that described in US20200107756A1, where light is internally generated by the radiation treatment beam in the tissue being probed.

Although the illustrations depict the light source 34 as a single light generating object, it will be appreciated that the light source 34 instead comprises a plurality of discrete light sources 34 which sequentially or simultaneously excite distinct quantized modes of excitation of interest, including but not limited to electronic, vibrational, and resonantly or non-resonantly vibronic. Notably, measurements performed in resonant mode (i.e., where the excitation source is tuned to specific frequency responses of the sample system), could be effective to enhance signal to noise considerations.

The wearable device 10 further includes an array of photodetectors 36 disposed in the housing. Each photodetector 36 comprises a discrete channel (λₙ) configured to detect photons entering the internal cavity through the port 24. The array comprises at least two photodetectors 36 (e.g., λ₁ and λ₂). Preferably, at least one photodetector is used to generate a reference signal and a plurality of photodetectors 36 are used to generate separate, i.e., discrete, channels for detecting multiple spectroscopic lines to capture changes in tissue optical parameters as needed.

In the illustrated examples, six photodetectors 36 (λ₁- λ₆) are strategically stationed in the internal cavity in a circular or annular pattern around the light source 34. Other arrangements of the photodetectors 36 are certainly possible, as may be determined beneficial by the designer. The photodetectors 36 are silicon photomultipliers (SiPM).

At least one optical filter is associated with each photodetector 36. The optical filter limits light reaching the associated photodetector 36 to a specific wavelength (lambda) and to eliminate Rayleigh scattered light. It is contemplated that the optical filter could be directly integrated with its associated photodetector 36, however in the illustrated examples the optical filter is shown as separate from the photodetector 36. Likewise, the optical filter could be a unitary element, but is depicted in Figures 5-7 as a first filter 38 and a second filter 40. The first filter 38 comprises a narrow band filter at a specific lambda, whereas the filter 40 comprises a spike filter to eliminate Rayleigh scattered light. Thus, the embodiment shown in the figures utilizes two filters 38, 40 for each channel (λₙ). The first filter 38 only passes a specific chosen wavelength (λ). The second filter 40 blocks any residual source light frequency. Typically, it does not matter in what order the light passes through the filters 38, 40 such that their arrangement relative to the associated photodetector 36 is subject to designer's choice. And as previously mentioned, the second filter 40 to eliminate Rayleigh scattered light could conceivably be a common filter serving all photodetectors 36, such as at the port 24 or some other convenient common location.

Optical filters suitable for use with the wearable device 10 may be selected from the group consisting essentially of bandpass, multi-bandpass, notch, edgepass, spike, Rayleigh scatter rejection, diffraction grating, Fabry-Perot interferometer, MEMs based interferometry, dye, and nano-photonic types, including combinations thereof. Optionally, light blocking paint (or epoxy, or other suitable coating or surface treatment) may be applied to the side edges of one or both filters 38, 40 according to known techniques. And as previously stated, one or both optical filters may be integrated with or spaced apart from the respective photodetector 36.

Preferably, a light shield 42 is disposed in the interior cavity between the light source 34 and the plurality of photodetectors 36. The purpose of the light shield 42 is to prevent, or at least reduce, light directly from the light source 34 or reflected from the surface of the skin 22 from reaching any of the photodetectors 36. Naturally, the light shield 42 can take many different forms. In the illustrated examples, the light shield 42 is shown surrounding the light source 34. However, alternative forms contemplate one or more light shields surrounding or otherwise partitioning the photodetectors 36. Considering the possibility for design variations, the light shield 42 shown in Figure 5 is a generally cylindrical, tubular structure that extends substantially from the underside of the cover 14 toward a terminal end adjacent the port 24. The terminal end of the light shield 42 is preferably disposed close to the surface of the skin 22 to maximize light blocking functionality. Optionally, the distal end of the light shield 42 can be made conformable or extendable to better perfect a light-tight seal against the skin 22. Figures 5 and 6 show in phantom a simple accordion-like member 44 biased downwardly that makes contact with the skin 22 and compresses when the wearable device 10 is lashed into place. Alternatively, the terminal end of the light shield 42 could be fitted with a flexible lip seal or a telescopic member. Many alternative configurations are possible if it is desired for the light shield to better perfect a light-tight seal against the skin 22. Moreover, if pane or membrane covers the port 24, suitable accommodations can be made for the accordion member 44 or extensible tip to pass through without sacrificing functionality.

The previously mentioned circuit board 32 preferably includes a suitable data acquisition electronics module capable of single photon counting over a predetermined sampling time. Sampling time can vary depending on the application. In some cases, the allotted sampling time will be in the range of 0 - 1000 ms. In other applications, allotted sampling times in the range of 1-10 seconds maybe sufficient. Furthermore, the data acquisition electronics module will preferably include a scalar or other functionality which digitally records the intensities of the specific Raman lines of interest, resulting from the excitation of specific quantized normal modes of molecular vibration. The quantized normal modes of vibration may include electronic modes, ultrasonic modes, acoustic modes and/or vibronic modes.

Having thus described the basic physical components of the wearable device 10, operation of the system can be understood in cooperation with Figure 7. Generally stated, by way of background, when light is scattered from a molecule or crystal, most photons are elastically scattered (i.e., unchanged in frequency). This is referred to as Rayleigh scattering. However, a small fraction of light (approximately 1 in 10⁷ photons) is scattered at optical frequencies different from, and usually lower than, the frequency of the incident photons. This inelastic process is known as Raman scattering and can occur with the excitation of a vibrational, rotational or electronic energy of a molecule. The vibrational excitations manifest in the spectrum of the scattered light as weak, Raman-shifted sidebands flanking the Rayleigh peak. Quantum considerations lead to upshifted and downshifted sidebands. Because the peaks that are downshifted in energy, referred to as the Stokes Raman spectrum, are generally more intense than the upshifted ones, measurements can be confined to the downshifted case. The Raman shift from particular vibrational modes, Δυ, is given (in wavenumbers) by the equation: $Δ ν = \frac{1}{{λ}_{i}} - \frac{1}{{λ}_{s}}$
where the subscripts, *i* and *s,* refer to the incident and scattered photons, respectively. This equation, essentially a statement of the conservation of energy, is germane to the present invention.

In Figure 7, the exemplary wearable device 10 is secured in position against the skin 22 of a user over a location of interest. The port 24 of the wearable device 10 is situated to enable the device 10 to interrogate subcutaneous molecules 46 in a non-invasive, real-time, continuous manner for the purpose of diagnosing a condition of interest. As previously mentioned, many different conditions of interest are contemplated. While one primary example may be taken as blood glucose monitoring, this is by no means the only possible application of the wearable device 10. Nevertheless, blood glucose monitoring serves as a good example through which to describe the operational characteristics of this invention. Blood glucose monitoring may be abbreviated a CGM, in reference to continuous glucose monitoring.

To obtain a signal that is unique to a subcutaneous molecular characteristic of interest, e.g., the glucose concentration in the blood, a Raman detector (λₛ) is tuned to satisfy the above equation for the particular vibrational frequency of the molecule of interest, e.g., the glucose molecule. This tuning step can be accomplished in a variety of ways. In one example, a first narrow-bandpass (spectral width ~10 nm) optical filter 38 can be placed directly on top of a high-gain (~10⁷) SiPM photodetector chip 36. An interference filter is convenient for this purpose, with the etalon (narrow band filter) selected to transmit light only at this wavelength. A second optical notch filter 40 can be used in combination with the first narrow-bandpass optical filter 38 to eliminate the much stronger Rayleigh scattered light from the Raman spectrum to ensure the signal detected is only from the Raman scattered light.

This elegant multispectral detection approach enables accurate readings of the subcutaneous molecular characteristic of interest, e.g., the blood glucose concentration directly by measuring the integrated intensity of the selected Raman active line relative to nearby Raman peaks associated with the tissue matrix such as water or hemoglobin, which can act as a reference. This technique simultaneously overcomes two barriers to miniaturization that have prevented the application of Raman spectroscopy to real-time glucose (or other molecular characteristic of interest) monitoring: (1) the spectroscopic bandpass filters 38 are each precisely tuned to one particular Raman peak, thus eliminating the need for a bulky scanning spectrometer; (2) the high-gain SiPM photodetector 36 is sensitive to weak Raman signals and is very compact (<2 mm²). The proposed design will incorporate a multi-channel array allowing for a reference signal and several separate channels for detecting multiple Raman lines to capture changes in tissue optical parameters as needed (e.g. skin 22 tone, skin 22 irritation, etc.). Contemplated arrays include 2x2, as well as the 6-channel array suggested by Figure 4. Of course, other array configurations are possible and may be preferred over the mentioned 2x2 and 6-channel arrays depending on the application.

The present invention may be understood to emphasize two aspects of Raman spectroscopy that are particularly relevant to the exemplary CGM application.

The first relates to the complex bioenvironment in which these measurements are performed: glucose molecules are immersed in blood/interstitial fluid with numerous vibrational modes principally associated with hemoglobin or water in the frequency range of interest. Note however that quantum selection rules limit Raman-active processes only to those in which the polarizability of the molecule is changed by the symmetry of the particular vibrational mode. This considerably reduces the complexity of the Raman spectrum to a few prominent and well-separated lines. For example, the Raman line of most interest here is the one at ~1125 cm⁻¹ (Figure 1) which is associated with the "breathing mode" of the glucose ring. Figure 1 shows other Raman lines of interest at about 572 cm⁻¹, 796 cm⁻¹, 1060 cm⁻¹ and 1360 cm⁻¹. The prominent hemoglobin Raman line at 1549 cm⁻¹ (not visible in Figure 1) is also of interest as a possible reference against which to calibrate the blood glucose concentration, along with Raman lines at about 436 cm⁻¹, 456 cm⁻¹, 527 cm⁻¹, 855 cm⁻¹, 912 cm⁻¹, 1060 cm⁻¹, 1366 cm⁻¹ and 1456 cm⁻¹. The idea of this ratiometric approach is to use the reference to remove any glucose Raman intensity variation due to changes other than glucose concentration in the blood. For example, if the wearable device 10 moves with respect to the measurement region of interest, the overall intensity may fluctuate but not the differential signal with respect to the reference. An effective lashing 28 will also serve to reduce or eliminate intensity variation provoked by relative movements between the wearable device 10 and the interrogated subcutaneous molecules 46. By also coordinating sampling with pulse measurements (i.e., sampling during a pulse and sampling in between pulses), blood sugar measurements can be delineated from interstitial fluid glucose measurements.

The second important physiological aspect is that the subcutaneous tissue and dermis that the incident light must traverse en route to the molecules 46 is turbid and has a fairly short absorption length (several mm). However, by judicious choice of light source 34 (e.g., laser or LED, excitation wavelength, λᵢ, etc.) the absorption of the incident and scattered light can be minimized considerably. One effective technique is to use red or near-infrared (NIR) light (λᵢ ≥660 nm) which propagates reasonably well (several cm) through soft tissue. As a further benefit, SiPM-type photodetectors 36 optimized for red light (the R-series) are commercially available. Thus, the inherent flexibility and selectivity of Raman spectroscopy can be leveraged effectively by the wearable device 10 to advance the design of a non-invasive, wearable, real-time, continuous diagnostic spectrometer and method for CGM as well as many other applications. The wearable device 10 of this invention can thus be configured as a continuous glucose monitor (CGM) to drastically improve the lives of diabetics and enhance health outcomes.

The extreme sensitivity and high gain of the described photodetector 36 technology is one key enabler of this innovation. The wearable device 10 utilizes the faint inelastic scattered light transmitted through tissue to enable spectral measurements of relevant molecular biomarkers. Inelastic scattered light is strongly absorbed and scattered by human tissue, leaving only a handful of measurable photons carrying information of interest, and only a subset of these within specific wavelengths are useful for molecular measurements. Similarly, an energy efficient light source 34 generates a very limited spectroscopically unique signal, necessitating a similarly sensitive and elegant technical approach. For the CGM application, the wearable device 10 employs Raman spectroscopy, targeting the tiny fraction of scattered photons that carry the "fingerprint" and concentration of the molecules of interest 46 in the blood or interstitial fluid. The wearable device 10 and its method of use uniquely overcome the intensity concerns of weak signal of interest which is greatly alleviated by the high sensitivity of the SiPM detector. In the application described in the present disclosure (biomolecules probed by Raman scattering) only certain components of the Raman scattered light are targeted, i.e., those possessing the relevant wavelengths necessary to measure the characteristic of interest (e.g., blood glucose), thereby eliminating the need for a physically large and expensive high-performance spectrometer and enabling a compact, wearable, and low-cost device.

The principles of this invention enable a truly noninvasive Raman probe in a wearable form factor. Through a uniquely new architecture, the wearable device 10 achieves tight integration of highly sensitive multichannel photodetectors 36 which incorporate high-gain photodetectors 36 coupled with narrow-band optical first filters 38 to enable measurement of specific Raman peaks of the molecule of interest 46. Furthermore, signal-to-noise in other Raman scattering applications is improved through miniaturizing the digital electronics that are needed for data acquisition, signal processing, and real time analysis, especially in photon counting mode. Multispectral data acquisition is important for quantitative analysis utilizing multivariate calibration models which have been shown to help achieve high quantitative accuracy based on measurement of several Raman lines, not just a single peak. The wearable device 10 brings the powerful and well-established Raman spectroscopic technique into the realm of practical utility as a wearable biomedical sensor technology. The invention serves not only as a truly noninvasive real time CGM, but also as a platform for measuring many other important physiological biomarkers.

In the specific example of CGM, the wearable device 10 represents a noninvasive approach to measuring blood glucose concentrations based on Raman spectroscopy. In one embodiment, monochromatic light (e.g., from a laser diode 34) is directed at subcutaneous tissue. The light generated scatters from the interstitial fluid and blood vessels. Some of this light also interacts with glucose molecules 46 in this matrix and excites vibrational modes specific to glucose. The frequencies of these characteristic modes (bond stretching, bending, etc.) are imprinted on the Raman-scattered light which, after exiting the body, is analyzed by an array of miniature narrow-band spectrometers 36. In this manner, Raman spectroscopy "fingerprints" the molecular bonds in chemistry and biology to provide a reliable quantitative probe of blood glucose concentration.

The foregoing invention has been described in accordance with the relevant legal standards, thus the description is exemplary rather than limiting in nature. Variations and modifications to the disclosed embodiment may become apparent to those skilled in the art and fall within the scope of the invention. Particular aspects of interest include:

With regard to the apparatus:
Wherein a light shield extends substantially from said light source toward a terminal end adjacent said port. In one form, the light shield is generally tubular and surrounds said light source.

The predetermined sampling time of the apparatus may be in the range of 0 - 1000 ms.The light source may be configured to produce monochromatic light having a frequency in the spectral band of 200nm - 1500nm. The light source may be configured to produce broadband light capable of activating fluorescence or phosphorescence responses from biological tissue being probed. The light source may be configured to produce tunable monochromatic light capable of performing measurements in resonant mode. The light source may comprise a plurality of discrete light sources each having a frequency in the spectral band of 200nm - 1500nm which simultaneously excite distinct quantized modes of excitation of interest.

## Claims

1. A non-invasive, wearable, diagnostic spectrometer apparatus (10) adapted for direct contact with a user's skin, comprising:
a housing having a sidewall surrounding an interior cavity, a cover (14) disposed over said sidewall and said interior cavity, a base (20) disposed under said sidewall at least partially enclosing said interior cavity, said base (20) having a port (24) therein configured to enable the transit of photons of light therethrough,
lashing (28) extending from said housing and configured to immobilize said interior cavity of said housing directly against the skin of a user at a region of interest,
a light source (34) configured to emit photons of light through said port (24) that will probe molecular biomarkers of physiological interest in subcutaneous tissue and return photons of Rayleigh scattered light commingled with Raman scattered light through said port (24) and into said interior cavity, said light source (34) comprising a plurality of discrete light emitting diodes (LEDs) each configured to produce monochromatic light having a wavelength in the spectral band of 200nm - 1500nm which sequentially or simultaneously excite distinct quantized modes of excitation of interest,
an array of photodetectors (36) disposed in said interior cavity of said housing, each photodetector (36) comprising a discrete channel (λₙ) configured to detect photons of Raman scattered light entering said interior cavity through said port (24), wherein said photodetectors (36) are silicon photomultipliers (SiPM),
at least one optical filter (38, 40) in said interior cavity and associated with each said SiPM photodetector (36), said optical filter (38, 40) operatively disposed between the associated said SiPM photodetector (36) and said port (24), each said optical filter (38, 40) limiting the transit of light reaching the associated said SiPM photodetector (36) to a specific wavelength and eliminating Rayleigh scattered light, and
a data acquisition electronics module fixed relative to said housing and operatively associated with said array of SiPM photodetectors (36), said data acquisition electronics module configured to count single photons of Raman scattered light reaching each said SiPM photodetector (36) over a predetermined sampling time.

2. The apparatus (10) of Claim 1, wherein array of photodetectors (36) comprises at least one SiPM photodetector (36) generating a reference signal and a plurality of SiPM photodetectors (36) detecting a plurality of discrete Raman lines.

3. The apparatus (10) of Claim 1, further including a light shield (42) disposed in said interior cavity between said light source (34) and said plurality of SiPM photodetectors (36).

4. The apparatus (10) of Claim 1, wherein said predetermined sampling time is in the range of 0-10 seconds.

5. The apparatus (10) of Claim 1, wherein said data acquisition electronics module is configured to count photons of the Raman scattered light resulting from the excitation of specific quantized normal modes of vibration, said quantized normal modes of vibration including at least one of optical vibrational modes, acoustic vibrational modes, ultrasonic modes and vibronic modes.

6. The apparatus (10) of Claim 1, wherein said optical filters (38, 40) are selected from the group consisting essentially of: bandpass, multi-bandpass, notch, edgepass, diffraction grating, and Fabry-Pérot interferometer.

7. The apparatus (10) of Claim 1, wherein each said optical filter (38, 40) comprises a first filter (40) configured to reject Rayleigh scattered light and a second filter (38) configured to limit the transit of light reaching the associated said SiPM photodetector (36) to a specific wavelength.

8. A method for non-invasively diagnosing a condition of subcutaneous biological tissue using Raman spectroscopy, said method comprising the steps of:
stationing a plurality of photodetectors (36) in an internal cavity having a port, the photodetectors (36) comprising silicon photomultipliers (SiPM), each SiPM photodetector comprising a discrete channel (λₙ) configured to detect photons of Raman scattered light entering the internal cavity through the port,
immobilizing the port of the interior cavity directly against the skin of a user,
emitting light from a light source (34) in the interior cavity through the port (24) and directly onto the skin of the user, wherein said step of emitting light includes producing monochromatic light having a wavelength in the spectral band of 200nm - 1500nm from a plurality of discrete light emitting diodes (LEDs) and wherein the LEDs sequentially or simultaneously excite distinct quantized modes,
interrogating with the light at least one subcutaneous molecule below the skin of the user, said interrogating step producing optical photons of Rayleigh scattered light commingled with Raman scattered light that re-enter the internal cavity through the port (24),
filtering the photons re-entering the internal cavity through the port (24) using at least one optical filter to eliminate Rayleigh scattered light,
filtering the Raman scattered light using at least one optical filter associated with each SiPM photodetector (36) to limit the Raman scattered light reaching each SiPM photodetector (36) to a specific wavelength associated with a Raman active line, and
counting single photons received in each SiPM photodetector (36) over a predetermined sampling time to measure the integrated intensity of the selected Raman active line.

9. The method of Claim 8 wherein the interrogated subcutaneous molecule below the skin of the user is selected from the group consisting essentially of: hemoglobin and glucose, and the selected Raman active line is selected from the group consisting essentially of about: 436 cm⁻¹, 456 cm⁻¹, 527 cm⁻¹, 572 cm⁻¹, 796 cm⁻¹, 855 cm⁻¹, 912 cm⁻¹, 1060 cm⁻¹, 1125 cm⁻¹, 1360 cm⁻¹, 1366 cm⁻¹, 1456 cm⁻¹, and 1549 cm⁻¹.

10. The method of Claim 8 wherein said step of emitting light from a light source (34) includes simultaneously exciting distinct quantized modes of excitation of interest selected from the group consisting essentially of: electronic, vibrational, and resonantly vibronic and non-resonantly vibronic.

11. The method of Claim 8 further including the step of transmitting data informed by the measured integrated intensity of the selected Raman active line to a remote computing device via a secure communication connection.

12. The method of Claim 8 further including the step of transmitting data informed by the measured integrated intensity of the selected Raman active line to a remote controller of a remedial device.

13. The method of Claim 8 further including the step of generating an alarm signal in response to the measured integrated intensity of the selected Raman active line.

## Patentansprüche

1. Nichtinvasive, tragbare diagnostische Spektrometervorrichtung (10), die für den direkten Kontakt mit der Haut eines Benutzers angepasst ist, umfassend:
ein Gehäuse mit einer Seitenwand, die eine Innenkammer umgibt, eine Abdeckung (14), die über der Seitenwand und der Innenkammer angeordnet ist, eine Basis (20), die unter der Seitenwand angeordnet ist, die die Innenkammer mindestens teilweise umschließt, wobei die Basis (20) eine darin ausgebildete Öffnung (24) aufweist, die so konfiguriert ist, dass sie den Durchtritt von Lichtphotonen durch diese ermöglicht,
ein Befestigungsband (28), das sich von dem Gehäuse erstreckt und so konfiguriert ist, dass es die Innenkammer des Gehäuses in einem interessierenden Bereich direkt an der Haut eines Benutzers fixiert,
eine Lichtquelle (34), die so konfiguriert ist, dass sie Lichtphotonen durch die Öffnung (24) aussendet, die molekulare Biomarker von physiologischem Interesse in subkutanem Gewebe untersucht, und Photonen von Rayleigh-gestreutem Licht, die mit Raman-gestreutem Licht vermischt sind, durch die Öffnung (24) und in die Innenkammer zurückführt, wobei die Lichtquelle (34) eine Vielzahl diskreter lichtemittierender Dioden (LEDs) umfasst, die jeweils so konfiguriert sind, dass sie monochromatisches Licht mit einer Wellenlänge im Spektralbereich von 200 nm bis 1500 nm erzeugen, wobei die LEDs nacheinander oder gleichzeitig verschiedene quantisierte Anregungsmoden von Interesse anregen,
eine Anordnung von Photodetektoren (36), die in der Innenkammer des Gehäuses angeordnet sind, wobei jeder Photodetektor (36) einen diskreten Kanal (λn) umfasst, der so konfiguriert ist, dass er Photonen von Raman-gestreutem Licht erfasst, die durch die Öffnung (24) in die Innenkammer eintreten, wobei die Photodetektoren (36) Silizium-Photomultiplier (SiPM) sind,
mindestens einen optischen Filter (38, 40) in der Innenkammer und verbunden mit jedem der SiPM-Photodetektoren (36), wobei der optische Filter (38, 40) wirkend zwischen dem verbundenen SiPM-Photodetektor (36) und der Öffnung (24) angeordnet ist, wobei jeder optische Filter (38, 40) den Durchtritt des Lichts, das den verbundenen SiPM-Photodetektor (36) erreicht, auf eine spezifische Wellenlänge begrenzt und Rayleigh-gestreutes Licht eliminiert, und
ein Datenerfassungselektronikmodul, das relativ zu dem Gehäuse befestigt und wirkend mit der Anordnung von SiPM-Photodetektoren (36) verbunden ist, wobei das Datenerfassungselektronikmodul so konfiguriert ist, dass es einzelne Photonen von Raman-gestreutem Licht, die jeden der SiPM-Photodetektoren (36) erreichen, über eine vorbestimmte Probenahmedauer zählt.

2. Vorrichtung (10) nach Anspruch 1, wobei die Anordnung von Photodetektoren (36) mindestens einen SiPM-Photodetektor (36), der ein Referenzsignal erzeugt, und eine Vielzahl von SiPM-Photodetektoren (36) umfasst, die eine Vielzahl diskreter Raman-Linien erfassen.

3. Vorrichtung (10) nach Anspruch 1, die ferner eine Lichtabschirmung (42) einschließt, die in der Innenkammer zwischen der Lichtquelle (34) und der Vielzahl von SiPM-Photodetektoren (36) angeordnet ist.

4. Vorrichtung (10) nach Anspruch 1, wobei die vorbestimmte Probenahmedauer im Bereich von 0 - 10 Sekunden liegt.

5. Vorrichtung (10) nach Anspruch 1, wobei das Datenerfassungselektronikmodul so konfiguriert ist, dass es Photonen des Raman-gestreuten Lichts zählt, die aus der Anregung spezifischer quantisierter Normalmoden von Schwingungen resultieren, wobei die quantisierten Normalmoden von Schwingungen mindestens eines aus Folgendem einschließen: optische Schwingungsmoden, akustische Schwingungsmoden, Ultraschallmoden und vibronische Moden.

6. Vorrichtung (10) nach Anspruch 1, wobei die optischen Filter (38, 40) im Wesentlichen ausgewählt sind aus der Gruppe bestehend aus Bandpassfiltern, Mehrfachbandpassfiltern, Bandsperrfiltern, Kantenpassfiltern, Beugungsgittern und Fabry-Pérot-Interferometern.

7. Vorrichtung (10) nach Anspruch 1, wobei jeder der optischen Filter (38, 40) einen ersten Filter (40) umfasst, der so konfiguriert ist, dass er Rayleigh-gestreutes Licht unterdrückt, und einen zweiten Filter (38) umfasst, der so konfiguriert ist, dass er den Durchtritt des Lichts, das den verbundenen SiPM-Photodetektor (36) erreicht, auf eine spezifische Wellenlänge begrenzt.

8. Verfahren zur nichtinvasiven Diagnose eines Zustands von subkutanem biologischem Gewebe unter Verwendung der Raman-Spektroskopie, wobei das Verfahren die folgenden Schritte umfasst:
Anordnen einer Vielzahl von Photodetektoren (36) in einer Innenkammer mit einer Öffnung, wobei die Photodetektoren (36) Silizium-Photomultiplier (SiPM) umfassen, wobei jeder SiPM-Photodetektor einen diskreten Kanal (λn) umfasst, der so konfiguriert ist, dass er Photonen von Raman-gestreutem Licht erfasst, die durch die Öffnung in die Innenkammer eintreten,
Fixieren der Öffnung der Innenkammer direkt an der Haut eines Benutzers,
Aussenden von Licht aus einer Lichtquelle (34) in der Innenkammer durch die Öffnung (24) und direkt auf die Haut des Benutzers, wobei der Schritt des Aussendens von Licht das Erzeugen von monochromatischem Licht mit einer Wellenlänge im Spektralbereich von 200 nm bis 1500 nm aus einer Vielzahl diskreter lichtemittierender Dioden (LEDs) umfasst, und wobei die LEDs nacheinander oder gleichzeitig verschiedene quantisierte Moden anregen,
Untersuchen mindestens eines subkutanen Moleküls unter der Haut des Benutzers mit dem Licht, wobei der Schritt des Untersuchens optische Photonen von Rayleigh-gestreutem Licht erzeugt, die mit Raman-gestreutem Licht vermischt sind und durch die Öffnung (24) wieder in die Innenkammer eintreten,
Filtern der durch die Öffnung (24) wieder in die Innenkammer eintretenden Photonen unter Verwendung mindestens eines optischen Filters, um Rayleigh-gestreutes Licht zu eliminieren,
Filtern des Raman-gestreuten Lichts unter Verwendung mindestens eines optischen Filters, der mit jedem SiPM-Photodetektor (36) verbunden ist, um das Raman-gestreute Licht, das die einzelnen SiPM-Photodetektoren (36) erreicht auf eine spezifische Wellenlänge zu begrenzen, die mit einer Raman-aktiven Linie verbunden ist, und
Zählen einzelner Photonen, die von jedem SiPM-Photodetektor (36) über eine vorbestimmte Probenahmedauer empfangen werden, um die integrierte Intensität der ausgewählten Raman-aktiven Linie zu messen.

9. Verfahren nach Anspruch 8, wobei das untersuchte subkutane Molekül unter der Haut des Benutzers im Wesentlichen ausgewählt ist aus der Gruppe bestehend aus Hämoglobin und Glukose und die ausgewählte Raman-aktive Linie ausgewählt ist aus der Gruppe im Wesentlichen bestehend aus: 436 cm⁻¹, 456 cm⁻¹, 527 cm⁻¹, 572 cm⁻¹, 796 cm⁻¹, 855 cm⁻¹, 912 cm⁻¹, 1060 cm⁻¹, 1125 cm⁻¹, 1360 cm⁻¹, 1366 cm⁻¹, 1456 cm⁻¹ und 1549 cm⁻¹.

10. Verfahren nach Anspruch 8, wobei der Schritt des Aussendens von Licht aus einer Lichtquelle (34) das gleichzeitige Anregen verschiedener quantisierter Anregungsmoden von Interesse einschließt, die im Wesentlichen ausgewählt sind aus der Gruppe bestehend aus: elektronischen, schwingungsbezogenen sowie resonant vibronischen und nicht resonant vibronischen Moden.

11. Verfahren nach Anspruch 8, das ferner den Schritt des Übertragens von Daten einschließt, die durch die gemessene integrierte Intensität der ausgewählten Raman-aktiven Linie an ein Remote-Computergerät über eine sichere Kommunikationsverbindung informiert sind.

12. Verfahren nach Anspruch 8, das ferner den Schritt des Übertragens von Daten einschließt, die durch die gemessene integrierte Intensität der ausgewählten Raman-aktiven Linie an eine Remote-Steuerung eines Abhilfegeräts informiert sind.

13. Verfahren nach Anspruch 8, das ferner den Schritt des Erzeugens eines Alarmsignals als Reaktion auf die gemessene integrierte Intensität der ausgewählten Raman-aktiven Linie einschließt.

## Revendications

1. Appareil de spectrométrie de diagnostic non invasif portable (10) apte à entrer en contact direct avec la peau d'un utilisateur, comprenant :
un boîtier présentant une paroi latérale entourant une cavité intérieure, un couvercle (14) disposé sur ladite paroi latérale et ladite cavité intérieure, une base (20) disposée sous ladite paroi latérale entourant au moins partiellement ladite cavité intérieure, ladite base (20) présentant un orifice (24) conçu pour permettre le passage des photons de lumière à travers elle,
une attache (28) s'étendant à partir dudit boîtier et conçue pour immobiliser ladite cavité intérieure dudit boîtier directement contre la peau d'un utilisateur au niveau d'une région d'intérêt,
une source de lumière (34) conçue pour émettre des photons de lumière à travers ledit orifice (24) qui sonderont des biomarqueurs moléculaires présentant un intérêt physiologique dans le tissu sous-cutané et renverront des photons de lumière diffusée Rayleigh mélangés à de la lumière diffusée Raman à travers ledit orifice (24) et dans ladite cavité intérieure, ladite source de lumière (34) comprenant une pluralité de diodes électroluminescentes (DEL) discrètes, chacune conçue pour produire une lumière monochromatique présentant une longueur d'onde dans la bande spectrale de 200 nm à 1500 nm qui excitent séquentiellement ou simultanément des modes quantifiés distincts d'excitation d'intérêt,
une matrice de photodétecteurs (36) disposés dans ladite cavité intérieure dudit boîtier, chaque photodétecteur (36) comprenant un canal discret (λn) conçu pour détecter des photons de lumière diffusée Raman entrant dans ladite cavité intérieure par ledit orifice (24), dans lequel lesdits photodétecteurs (36) sont des photomultiplicateurs en silicium (SiPM),
au moins un filtre optique (38, 40) dans ladite cavité intérieure et associé à chacun desdits photodétecteurs à SiPM (36), ledit filtre optique (38, 40) étant disposé de manière fonctionnelle entre ledit photodétecteur à SiPM associé (36) et ledit port (24), chaque filtre optique (38, 40) limitant le passage de la lumière atteignant ledit photodétecteur à SiPM associé (36) à une longueur d'onde spécifique et éliminant la lumière diffusée Rayleigh, et
un module électronique d'acquisition de données fixe par rapport audit boîtier et associé de manière fonctionnelle à ladite matrice de photodétecteurs à SiPM (36), ledit module électronique d'acquisition de données étant conçu pour compter des photons uniques de lumière diffusée Raman atteignant chacun desdits photodétecteurs à SiPM (36) pendant un temps d'échantillonnage prédéfini.

2. Appareil (10) selon la revendication 1, dans lequel la matrice de photodétecteurs (36) comprend au moins un photodétecteur à SiPM (36) générant un signal de référence et une pluralité de photodétecteurs à SiPM (36) détectant une pluralité de lignes Raman discrètes.

3. Appareil (10) selon la revendication 1, comportant en outre un écran de protection contre la lumière (42) disposé dans ladite cavité intérieure entre ladite source de lumière (34) et ladite pluralité de photodétecteurs à SiPM (36).

4. Appareil (10) selon la revendication 1, dans lequel ledit temps d'échantillonnage prédéfini est dans une plage comprise entre 0 et 10 secondes.

5. Appareil (10) selon la revendication 1, dans lequel ledit module électronique d'acquisition de données est conçu pour compter des photons de la lumière diffusée Raman résultant de l'excitation de modes normaux quantifiés spécifiques de vibration, lesdits modes normaux quantifiés de vibration comportant au moins l'un parmi les modes vibratoires optiques, les modes vibratoires acoustiques, les modes ultrasoniques et les modes vibroniques.

6. Appareil (10) selon la revendication 1, dans lequel lesdits filtres optiques (38, 40) sont choisis dans le groupe constitué essentiellement de : filtre passe-bande, filtre passe-bande multiple, filtre coupe-bande/réjecteur, filtre à front, réseau de diffraction et interféromètre Fabry-Pérot.

7. Appareil (10) selon la revendication 1, dans lequel chacun desdits filtres optiques (38, 40) comprend un premier filtre (40) conçu pour rejeter la lumière diffusée Rayleigh et un second filtre (38) conçu pour limiter le passage de la lumière atteignant ledit photodétecteur à SiPM associé (36) à une longueur d'onde spécifique.

8. Procédé de diagnostic non invasif d'un état de tissu biologique sous-cutané à l'aide de la spectroscopie Raman, ledit procédé comprenant les étapes consistant à :
placer une pluralité de photodétecteurs (36) dans une cavité interne présentant un orifice, les photodétecteurs (36) comprenant des photomultiplicateurs en silicium (SiPM), chaque photodétecteur à SiPM comprenant un canal discret (λn) conçu pour détecter les photons de la lumière diffusée Raman entrant dans la cavité interne par l'orifice,
immobiliser l'orifice de la cavité intérieure directement contre la peau d'un utilisateur,
émettre de la lumière à partir d'une source de lumière (34) dans la cavité intérieure à travers l'orifice (24) et directement sur la peau de l'utilisateur, dans lequel ladite étape d'émission de lumière comporte la production de lumière monochromatique présentant une longueur d'onde dans la bande spectrale de 200 nm à 1500 nm à partir d'une pluralité de diodes électroluminescentes discrètes (DEL) et dans lequel les DEL excitent séquentiellement ou simultanément des modes quantifiés distincts,
interroger avec la lumière au moins une molécule sous-cutanée sous la peau de l'utilisateur, ladite étape d'interrogation produisant des photons optiques de lumière diffusée Rayleigh mélangés à la lumière diffusée Raman qui rentrent dans la cavité interne par l'orifice (24),
filtrer les photons rentrant dans la cavité interne par l'orifice (24) à l'aide d'au moins un filtre optique pour éliminer la lumière diffusée Rayleigh,
filtrer la lumière diffusée Raman à l'aide d'au moins un filtre optique associé à chaque photodétecteur à SiPM (36) afin de limiter la lumière diffusée Raman atteignant chaque photodétecteur à SiPM (36) à une longueur d'onde spécifique associée à une ligne active Raman, et
compter les photons uniques reçus dans chaque photodétecteur à SiPM (36) pendant un temps d'échantillonnage prédéfini afin de mesurer l'intensité intégrée de la ligne active Raman choisie.

9. Procédé selon la revendication 8, dans lequel la molécule sous-cutanée interrogée sous la peau de l'utilisateur est choisie dans le groupe constitué essentiellement par : l'hémoglobine et le glucose, et la ligne active Raman choisie est choisie dans le groupe constitué essentiellement par environ : 436 cm⁻¹, 456 cm⁻¹, 527 cm⁻¹, 572 cm⁻¹, 796 cm⁻¹, 855 cm⁻¹, 912 cm⁻¹, 1060 cm⁻¹, 1125 cm⁻¹, 1360 cm⁻¹, 1366 cm⁻¹, 1456 cm⁻¹ et 1549 cm⁻¹.

10. Procédé selon la revendication 8, dans lequel ladite étape d'émission de lumière à partir d'une source de lumière (34) comporte l'excitation simultanée de modes quantifiés distincts d'excitation d'intérêt choisis dans le groupe constitué essentiellement par : électronique, vibratoire, vibronique résonant et vibronique non résonant.

11. Procédé selon la revendication 8, comportant en outre l'étape de transmission de données informées par l'intensité intégrée mesurée de la ligne active Raman choisie à un dispositif informatique distant par l'intermédiaire d'une connexion de communication sécurisée.

12. Procédé selon la revendication 8, comportant en outre l'étape de transmission de données informées par l'intensité intégrée mesurée de la ligne active Raman choisie à un contrôleur distant d'un dispositif correcteur.

13. Procédé selon la revendication 8, comportant en outre l'étape de génération d'un signal d'alarme en réponse à l'intensité intégrée mesurée de la ligne active Raman choisie.
